Europäisches Patentamt

European Patent Office

Office européen des brevets

(19)

(11) **EP 1 003 420 B1**

(12) **EUROPÄISCHE PATENTSCHRIFT**

(45) Veröffentlichungstag und Bekanntmachung des Hinweises auf die Patenterteilung:
**25.02.2004 Patentblatt 2004/09**

(21) Anmeldenummer: **98941386.9**

(22) Anmeldetag: **21.07.1998**

(51) Int Cl.⁷: $A61B\ 6/14$, $A61B\ 6/02$

(86) Internationale Anmeldenummer:
**PCT/EP1998/004538**

(87) Internationale Veröffentlichungsnummer:
**WO 1999/004692 (04.02.1999 Gazette 1999/05)**

(54) **RÖNTGENDIAGNOSTIKGERÄT FÜR TOMOSYNTHESE**

X-RAY EXAMINATION UNIT FOR TOMOSYNTHESIS

APPAREIL DE DIAGNOSTIC AUX RAYONS X DESTINE A LA TOMOSYNTHESE

(84) Benannte Vertragsstaaten:
**DE FI FR GB IT**

(30) Priorität: **24.07.1997 DE 19731927**

(43) Veröffentlichungstag der Anmeldung:
**31.05.2000 Patentblatt 2000/22**

(73) Patentinhaber: **Sirona Dental Systems GmbH
64625 Bensheim (DE)**

(72) Erfinder: **PLÖTZ, Josef
D-81669 München (DE)**

(74) Vertreter: **Sommer, Peter
Sommer
Patentanwalt und
European Patent and Trademark Attorney
Viktoriastrasse 28
68165 Mannheim (DE)**

(56) Entgegenhaltungen:
**EP-A- 0 632 995        DE-A- 3 028 996
DE-A- 3 215 280**

**Beschreibung**

Die Erfindung betrifft ein Verfahren zum Betrieb und eine Vorrichtung zum Erzeugen von Tomosyntheseaufnahmen mittels eines Röntgendiagnostikgerätes.

Hierbei bzw. hierzu wird ein Untersuchungsobjekt aus unterschiedlichen Projektionsrichtungen durchstrahlt und der Strahlenschatten auf einen Strahlenempfänger abgebildet. Insbesondere bei Strahlenempfängern zum Erzeugen von elektrischen Signalen in Abhängigkeit vom auftreffenden Strahlenschatten können mit bekannten Rechenverfahren Schichtaufnahmen oder dreidimensionale Bilder errechnet werden. Aus der gewünschten Tiefenauflösung der Schichtdicke eines Bildes ergibt sich die Anzahl der nötigen Aufnahmen und der Raumwinkel der Durchstrahlung.

Bei konventionellen Schichtaufnahmen (Tomographie) werden ein Strahlensender und ein Strahlenempfänger gekoppelt und gegenläufig relativ zueinander verstellt. Objekte, die in der Fokalebene liegen, werden scharf abgebildet, da sie während der gegenläufigen Verstellung immer auf die gleiche Stelle des Strahlenempfängers projiziert werden. Objekte, die außerhalb der Fokalebene angeordnet sind, werden unscharf abgebildet, da sie während der gegenläufigen Verstellung auf unterschiedliche Stellen des Strahlenempfängers projiziert werden. Zur Erstellung einer auswertbaren Aufnahme wird das in der Fokalebene liegende Objekt durch mehrere Einzelprojektionen unter unterschiedlichen Projektionswinkeln $\alpha$ auf den Strahlenempfänger abgebildet. Durch direkte Überlagerung der durch die Einzelprojektionen gewonnenen Durchstrahlungsbilder ist ein tomographisches Bild des Objektes in der Fokalebene erstellbar. Ein tomographisches Bild eines Objektes, das in einer zur Fokalebene parallelen Ebene angeordnet ist, kann dadurch erstellt werden, daß die durch die Einzelprojektionen gewonnenen Durchstrahlungsbilder vor der Überlagerung um Strecken $\Delta S$ zueinander verschoben werden. Die Größe und die Richtung der Verschiebung $\Delta S$ hängt von der Position des Strahlensenders und von der Lage der zu rekonstruierenden Ebene ab.

Die Verschiebung $\Delta S$ ergibt sich für die sogenannte lineare Tomographie, bei welcher der Strahlensender in einer Dimension verstellt wird, durch die Formel:

$$\Delta S = \frac{x \cdot h}{x - y - h} \tan \alpha$$

wobei:

X = Abstand des Fokusses des Strahlensenders zum Strahlenempfänger,

h = Abstand der Fokalebene zur Ebene, in der ein Objekt rekonstruiert werden soll,

Y = Abstand der Fokalebene zur Ebene des Strahlenempfängers und

$\alpha$ = Projektionswinkel, d.h. der Winkel, den ein Bezugsstrahl des Strahlenbündels zu einer Bezugsachse einnimmt, wobei die Bezugsachse senkrecht zur Fokalebene ausgerichtet ist.

Buch Bildgebende Systeme für die medizinische Diagnostik, "Tomosynthese", Herausgeber Erich Krestel, Verlag Siemens Berlin/München, 2. Auflage, 1988, Seiten 380 und 381.

Wandelt der Strahlenempfänger den empfangenen Strahlenschatten des Objektes in elektrische Signale, so ermöglicht die digitale Tomosynthese die Rekonstruktion tomographischer Bilder in mehreren Ebenen aus den Signalen der Einzelprojektionen des Objektes, die bei unterschiedlichem Projektionswinkel $\alpha$ erstellt wurden. Bei der digitalen Tomosynthese können zur Erstellung eines sichtbaren Bildes aus den Signalen des Strahlenempfängers bekannte, digitale Bilderzeugungs- und Verarbeitungssysteme Anwendung finden.

Aus der WO 93/22 893 A1 ist eine Methode bekannt, mit der es möglich ist, eine Aufnahme eines Objektes zu rekonstruieren, ohne daß hierbei die Projektionswinkel $\alpha$ und die geometrische Anordnung von Strahlensender, Strahlenempfänger und Fokalebene bekannt sind. Gemäß dieser Methode ist im Bereich des Strahlenempfängers eine Referenz aus Strahlung absorbierendem Material mit bekannter Größe und bekanntem Abstand zum Strahlenempfänger vorgesehen, die bei jeder Einzelprojektion auf den Strahlenempfänger projiziert wird. Aufgrund der örtlichen Abbildung der Referenz auf den Strahlenempfänger für jede Einzelprojektion kann die geometrische Anordnung und der zweidimensionale Projektionswinkel $\alpha$ ermittelt werden. Diese Rekonstruktion ist aufgrund der umfangreichen Berechnungen zeitintensiv und aufwendig.

Zum Erhalt einer tomosynthetisch auswertbaren Bildfolge muß der Strahlensender vorgegebene Positionen und Ausrichtungen zum Untersuchungsobjekt einnehmen. Die Ausrichtung kann z.B. durch einen Bediener der Röntgendiagnostikeinrichtung oder durch die Verwendung und Ansteuerung eines Strahlensenders erfolgen, der mehrere Foki besitzt.

Aus der US-PS 5,598,454 sowie der WO 93/22893 A1 sind Röntgendiagnostikeinrichtungen für Tomosyntheseaufnahmen bekannt.

Die aus DE-A-3 028 996 bekannte Vorrichtung zur Tomosynthese weist mehrere in einer Strahlenquellenebene liegende Strahlenquellen auf. Diese Mehrfach Strahlenquelle wird so oft wie nötig kurzzeitig eingeschaltet, während die Teil perspektivbilder von den Bilddetektoren aufgenommen und auschließend abgespeichert werden.

Aus der EP 0 632 995 A1 ist eine zahnärztliche Röntgendiagnostikeinrichtung bekannt, bei der unter Verwendung eines Panorama-Aufnahmegerätes mit einem Röntgenstrahler und einer diametral gegenüber dem Röntgenstrahler angeordneten Aufnahmeeinheit auch tomosynthetische Aufnahmen von Objekten erstellt werden können. Hinsichtlich des Aufbaues herkömmlicher Panorama-Röntgenaufnahmevorrichtungen sowie Vorrichtungen zur Erstellung von Schädelaufnahmen wird auf die EP 0 229 308 A1 verwiesen. Die Erstellung einer Panorama-Aufnahme erfolgt in der Weise, daß bei der Strahlenabtastung des aufzunehmenden Objektes (Kiefer) gewonnenen Signale in einem zweidimensional auflösenden Detektor aufaddiert werden, wobei das Aufaddieren der Signale, falls ein CCD-Sensor verwendet wird, bereits durch den Sensor durchgeführt werden kann, indem dieser im TDI-Modus betrieben wird. Durch diese besondere Betriebsart wird die Funktion eines bewegten Filmes nachgebildet, in dem die durch Belichtung erzeugten Ladungspakete im CCD-Element entsprechend weitergetaktet werden, während ständig neue Ladungen hinzukommen. Die Taktimpulse für den TDI-Betrieb werden aus dem sonst für den Filmkassettenantrieb erforderlichen Schrittmotorimpulsen abgeleitet. Ferner kann alternativ auch ein Aufaddieren der Signale in einer späteren Signalverarbeitungsstufe möglich sein.

Aus den vom CCD-Sensor aus unterschiedlichen Durchstrahlungsrichtungen ableitbaren, somit gewonnenen Signalen, können Tomosyntheseaufnahmen erstellt werden. Unterwirft man die Signale nämlich tomosynthetischen Rekonstruktionsalgorithmen, statt sie gemäß den TDI-Takten aufzuaddieren, können scharfe

Schichten mit unterschiedlicher, nachträglich bestimmbarer Lage erzeugt werden. Hierbei müssen jedoch ernorm hohe Datenraten und Datenmengen in Kauf genommen werden.

Ein Ziel der vorliegenden Erfindung ist es, diese Nachteile zu vermeiden und mit nur einer Strahlenabtastung, mit einer einem normalen Panoramaröntgengerät entsprechenden Bewegungsmechanik und gut handhabbaren Datenraten und Datenmengen mehrere nachträglich bestimmbare scharfe Bildschichten erzeugen zu können. Ein weiteres Ziel der vorliegenden Erfindung ist es, ohne die Entwicklung neuer, spezieller CCD-Strahlendetektoren auszukommen, sondern statt dessen heute bereits verfügbare bzw. bei Panoramaröntgengeräten eingesetzte CCD-Strahlendetektoren verwenden zu können.

Die Aufgabe wird erfindungsgemäß durch den Gegenstand des Patentanspruches 1 und 7 gelöst.

Vorteil der Erfindung ist, daß Strahlungsimpulse während der Strahlungsabtastung eines Untersuchungsobjektes aus unterschiedlichen Projektionsrichtung erzeugt werden und daß die bei der Strahlenabtastung vom Festkörperstrahlenempfänger ableitbaren Signale während der Strahlungspause ausgelesen werden, wodurch das Auslesen von der mechanischen Verstellung der Aufnahmeeinheit aus Strahlensender und Strahlenempfänger entkoppelt ist, kein TDI-Betrieb mehr nötig ist und wodurch die somit ableitbaren Signale keinen "Verwischungsanteil" mehr haben, der beinhaltet wäre, wenn Strahlung während der Abtastung erzeugt und die Aufnahmeeinheit hierbei verstellt werden würde. Zudem können hierbei bereits verfügbare und bekannte CCD-Strahlungswandler Anwendung finden.

Besonders vorteilhaft ist, wenn die Strahlenabtastung unter schrittweiser Veränderung der Projektionsrichtungen erfolgt, wenn bei einer ersten Projektionsrichtung ein Strahlungsimpuls erzeugt und wenn während der Verstellung in eine zweite Projektionsrichtung während der Strahlungspause die Signale des Festkörperstrahlenempfängers ausgelesen werden. Die Datenmenge ist somit reduziert, da während der Strahlungspause und während der Verstellung in die zweite Projektionsrichtung keine Bildsignale erzeugt werden.

Zur Reduzierung der Unschärfeeffekte ist es vorteilhaft, wenn die Geschwindigkeit der Verstellung der Aufnahmeeinheit aus Strahlensender und Strahlenempfänger während der Strahlungsimpulse geringer ist, als während der Strahlungspausen.

Erfolgt die Strahlenabtastung des Objektes abschnittsweise, so kann die Berechnung eines Schichtbildes für einen Abschnitt bereits durchgeführt werden, während für zumindest einen weiteren Abschnitt die Strahlenabtastung noch erfolgt.

Weitere Vorteile und Einzelheiten der Erfindung ergeben sich aus der nachfolgenden Beschreibung eines Ausführungsbeispiels anhand der Zeichnungen in Verbindung mit den Unteransprüchen.

Es zeigt:

FIG 1 eine Röntgendiagnostikeinrichtung nach der Erfindung in einer prinzipiellen Darstellung und

FIG 2 ein Diagramm der Strahlungsimpulse und Strahlungspausen.

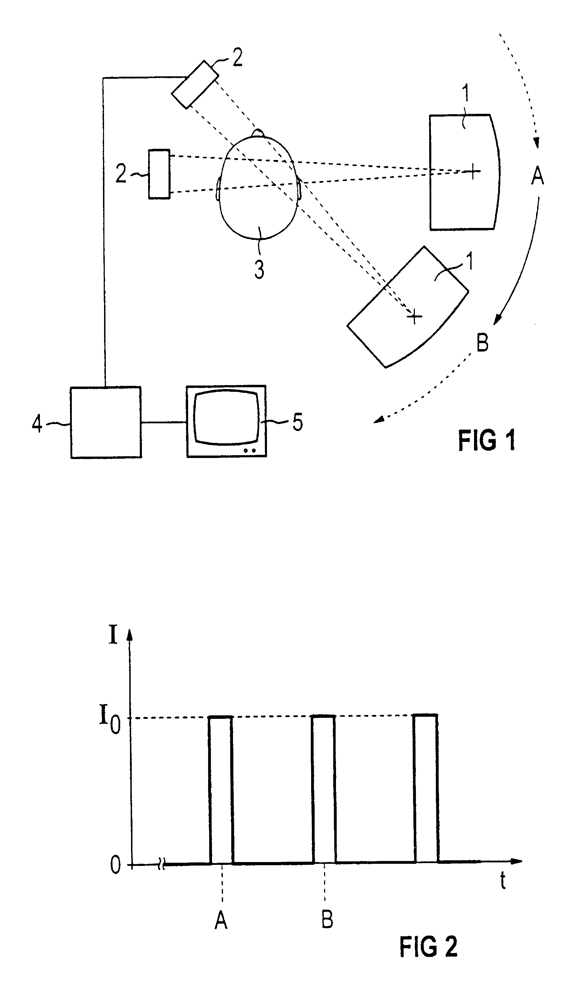

Beim Gegenstand der vorliegenden Erfindung kann eine Röntgendiagnostikeinrichtung Anwendung finden, bei der ein Strahlenempfänger zum Erzeugen von elektrischen Signalen, vorzugsweise ein Festkörperstrahlendetektor, Verwendung findet und mit der es möglich ist, ein Objekt mit Strahlung abzutasten. Deshalb ist in der FIG 1 in nur prinzipieller Weise eine Röntgendiagnostikeinrichtung gezeigt, die einen Strahlensender 1 und einen Strahlenempfänger 2 aufweist, die Teil einer Aufnahmeeinrichtung sind. Der Strahlensender 1 und der Strahlenempfänger 2 sind einander gegenüberliegend angeordnet und haben einen festen Bezug zueinander. Über eine nicht näher dargestellte Verstelleinrichtung sind sie um ein Objekt 3 verstellbar. Die Verstellung erfolgt hierbei durch Ansteuerung über eine Rechen- und Steuereinrichtung 4, die auch den Strahlensender 1 hinsichtlich des Erzeugens von Strahlungsimpulsen ansteuert. Die Signale des Strahlenempfängers 2 werden dieser Rechen- und Steuereinrichtung 4 zugeführt, die zur Berechnung von Schichtaufnahmen ausgebildet ist und Signale erzeugt, so daß auf einer der Rechen- und Steuereinrichtung 4 nachgeschalteten Anzeigeeinrichtung 5, insbesondere Tomosyntheseschichtaufnahmen, dargestellt werden können.

Aufgrund der somit vom Strahlenempfänger 2 ableitbaren Signale können Einzelbilder in Schichtlagen berechnet werden, die somit keine Bewegungsunschärfen mehr aufweisen, diese zumindest aber reduziert sind.

Ferner kann die Impulsdauer während der Strahlung verlängert und die Intensität reduziert werden, die verkürzt bzw. erhöht werden müßte, wenn die Verstellung der Aufnahmeeinheit kontinuierlich erfolgen würde.

Vorzugsweise erfolgt die Ansteuerung des Strahlensenders 2 und der Verstelleinrichtung derart, daß jeder der unterschiedlichen Projektionsrichtungen jeweils ein Strahlungsimpuls zugeordnet wird.

Die Verstellung kann hierbei kontinuierlich erfolgen, wobei es jedoch als vorteilhaft anzusehen ist, wenn die Geschwindigkeit der Verstellung während eines vom Strahlensender 1 ausgehenden Strahlungsimpulses zumindest geringer ist, als während der Strahlungspausen. Vorzugsweise wird die Verstellung während des Strahlungsimpulses angehalten und während der Strahlenpause fortgeführt.

Wie bereits erläutert, erfolgt die Strahlenabtastung vorzugsweise unter schrittweiser Veränderung der Projektionsrichtungen, wobei bei einer ersten Projektionsposition A ein Strahlungsimpuls erzeugt und wobei während der Verstellung in eine zweite Projektionsposition B während der Strahlungspause die Signale des Strahlenempfängers 2 ausgelesen werden. Es ist somit nicht mehr notwendig den Strahlenempfänger 2 im TDI-Betrieb, in dem eine Aufintegration erfolgt, zu betreiben, da das Auslesen während der Verstellung der Aufnahmeeinheit, aber bei abgeschalteter Strahlung erfolgt. Da die Signale durch einen Strahlungsimpuls erzeugt werden, bei dem die Aufnahmeeinrichtung vorzugsweise ortsfest ist, zumindest aber mit gegenüber der Strahlungspause verminderten Geschwindigkeit verstellt wird, enthalten die Signale des Strahlenempfängers 2 und damit die berechenbaren Einzelbilder keinen Verwischungsanteil mehr.

Findet als Verstelleinrichtung ein Schrittmotor Anwendung, so kann beispielsweise gegenüber dem Stand der Technik, wo im TDI-Betrieb eine kontinuierliche, d.h. taktgesteuerte Abtastung des Objektes 3 erfolgt, lediglich alle zehn Bewegungstakte ein Strahlungsimpuls erzeugt werden, wodurch bei einem z.B. 6 mm breiten CCD-Strahlungswandler und einer Pixelgröße von 0,1 mm eine 6-fache Datenmenge und bei gleicher Schrittmotorfrequenz wie im TDI-Betrieb auch nur eine 6-fache Datenrate erhalten wird. Der Spitzenwert der Datenrate verringert sich aber noch um ca. das 2,5-fache, wenn alle Verstellphasen mit der gleichen Geschwindigkeit erfolgen, was durch die Entkopplung vom Bildaufbau möglich ist. Wegen der relativ niedrigen Datenraten sind auch die Störeinflüsse und Rauschbeiträge, die vom CCD-Strahlungswandler und von der Ausleseelektronik ausgehen, gering, so daß kein signifikanter Dosismehrbedarf erforderlich ist. Diese Ausführungen gelten natürlich sinngemäß auch für beliebige andere Ausgestaltungen der Verstelleinrichtung.

Zur Verkürzung der Strahlenabtastung des Objektes 3 können Strahlungsimpulse hoher Intensität aber kurzer Zeitdauer Anwendung finden. Beispielsweise könnten zur Signalerzeugung Strahlungsimpulse eine Dauer von 20 bis 30 ms und Strahlungsimpulspausen, d.h. die Auslesezeit, eine Dauer von ca. 50 ms haben. Bei einer Abtastung des Objektes 3, die beispielsweise eine Zeit von 20 s in Anspruch nimmt, könnten Signale für 300 Einzelbilder erzeugt werden, die zusammen ca. 30 MB Speicherplatzbedarf haben.

Die Strahlungsimpulse des Strahlensender 1 können durch entsprechende Ansteuerung des Strahlengenerators oder durch Steuerung eines elektromechanischen Strahlenverschlusses erzeugt werden. Als Strahlenempfänger 2 können CCD-Strahlungswandler mit einer Szintillatorschicht oder aber auch aSi-, aSe- oder CdTe-Detektoren Anwendung finden.

Als vorteilhaft kann es sich auch erweisen, wenn bei einer kontinuierlichen Verstellung der Aufnahmeeinheit eine Belichtung mit sehr kurzen Strahlungsimpulsen erfolgt oder während einer normalen, schnellen oder langsamen Verstellgeschwindigkeit eine teilweise TDI-Vorintegration den Signalen beigemischt wird.

Um den Zeitbedarf für die Erstellung einer Tomosyntheseaufnahme zu verkürzen ist es vorteilhaft, wenn die Strahlenabtastung abschnittsweise erfolgt, wobei die Berechnung eines Schichtbildes für einen Abschnitt bereits durchgeführt wird, während für zumindest einen weiteren Abschnitt die Strahlenabtastung noch erfolgt.

**Patentansprüche**

1.   Verfahren zum Betrieb eines Röntgendiagnostikgerätes für digitale Tomosynthese aufweisend die Verfahrensschritte:

    a) Erzeugen von Strahlungsimpulsen während der Strahlenabtastung eines Objektes 3 aus unterschiedlichen Projektionsrichtungen,
    b) Auslesen der bei der Strahlenabtastung vom Strahlenschatten des Objektes (3) erzeugten und von einem Strahlenempfänger (2) abgeleiteten elektrischen Signale während der Strahlungspause,
    c) Zuführen dieser Signale zu einer Rechen- und Steuereinrichtung (4) und
    d) Berechnen zumindest einer Schichtaufnahme in zumindest einer vorbestimmbaren Schichtlage aufgrund dieser Signale.

**2.** Verfahren nach Anspruch 1,
wobei die Strahlungsimpulse durch Ansteuerung eines Strahlengenerators oder durch Ansteuerung einer Strahlenunterbrechungseinrichtung erzeugt werden.

**3.** Verfahren nach Anspruch 1 oder 2,
wobei die Strahlenabtastung unter schrittweiser Veränderung der Projektionsrichtungen erfolgt,
wobei bei einer ersten Projektionsrichtung ein Strahlungsimpuls erzeugt und
wobei während der Verstellung in eine zweite Projektionsrichtung während der Strahlungspause die Signale des Strahlenempfängers (2) ausgelesen werden.

**4.** Verfahren nach Anspruch 3,
wobei die Geschwindigkeit der Verstellung einer Aufnahmeeinheit aus Strahlensender (1) und Strahlenempfänger (2) des Röntgendiagnostikgerätes während der Strahlungsimpulse geringer ist als während der Strahlungspause.

**5.** Verfahren nach einem der Ansprüche 1 bis 4,
wobei die Strahlenabtastung abschnittsweise erfolgt und
wobei die Berechnung eines Schichtbildes für einen Abschnitt bereits durchgeführt wird, während für zumindest einen weiteren Abschnitt die Strahlenabtastung noch erfolgt.

**6.** Verfahren nach einem der Ansprüche 1 bis 5,
wobei der Strahlenempfänger (2) als CCD-, aSi-, aSe- oder als CdTe-Detektor ausgeführt ist.

**7.** Röntgendiagnostikgerät für digitale Tomosynthese, enthaltend

- einen Strahlensender (1),
- einen Strahlenempfänger (2),
- eine Verstelleinrichtung zur Verstellung von Strahlensender (1) und Strahlenempfänger (2),
- sowie eine Rechen- und Steuereinrichtung (4), die den Strahlensender (1) und die Verstelleinrichtung ansteuert, der vom Strahlenempfänger (2) abgeleitete elektrische Signale zugeführt werden, und die zur Berechnung von Schichtaufnahmen ausgebildet ist,

**dadurch gekennzeichnet, daß** die Rechen- und Steuereinrichtung in einer solchen Weise ausgebildet ist,

- daß sie den Strahlensender so ansteuert, daß dieser Strahlungsimpulse erzeugt,
- daß sie die vom Strahlenempfänger abgeleiteten elektrischen Signale während der Strahlungspause ausliest,

- und daß sie eine Schichtaufnahme in zumindest einer vorbestimmbaren Schichtlage aufgrund dieser Signale berechnet.

**Claims**

**1.** A method of operating a roentgendiagnostic appliance for digital tomosynthesis comprising the following steps:

a) generating radiation pulses during irradiation scanning of an object (3) from different directions of projection,

b) reading, during the pause between pulses, the electric signals produced by the radiation shadow of the object (3) during irradiation scanning and detected by a radiation detector (2),

c) feeding said signals to a computing controller (4) and

d) computing at least one tomographic image in at least one predeterminable layer on the basis of said signals.

**2.** A method as defined in claim 1, wherein the radiation pulses are produced by actuation of a radiation generator or by actuation of a radiation interceptor.

**3.** A method as defined in claim 1 or claim 2, wherein irradiation scanning is carried out using stepwise alteration of the directions of projection, a radiation pulse being produced in a first direction of projection whilst the signals coming from the detector (2) are being read during the pause between pulses occuring when shifting to a second direction of projection.

**4.** A method as defined in claim 3, wherein the velocity of adjustment of an X-ray unit, comprising a radiation emitter (1) and a radiation detector (2), of the roentgendiagnostic appliance is less during the radiation pulses than during the pause between said pulses.

**5.** A method as defined in any one of claims 1 to 4, wherein irradiation scanning is carried out intermittently and computation of a tomogram for one phase is carried to completion while irradiation scanning is still being carried out for at least one other phase.

**6.** A method as defined in any one of claims 1 to 5, wherein the radiation detector (2) is in the form of a CCD, aSi, aSe or CdTe detector.

**7.** A roentgendiagnostic appliance for digital tomosynthesis, comprising

- a radiation emitter (1),
- a radiation detector (2),
- adjusting means for the adjustment of said radiation emitter (1) and radiation detector (2), and
- a computing controller (4) adapted to actuate said radiation emitter (1) and said adjusting means, to which computing controller (4) adapted for computation of tomographic images there are fed the electric signals coming from the radiation detector (2),

**characterized in that** the computing controller is designed in such a manner that it

- actuates the radiation emitter to cause the latter to produce radiation pulses,
- reads the electric signals coming from the radiation detector during the pause between pulses, and
- computes a tomographic image in at least one predeterminable layer on the basis of said signals.

## Revendications

1. Procédé d'exploitation d'un appareil de diagnostic radiographique pour la tomosynthèse numérique, présentant les étapes opératoires suivantes :

    a) création d'impulsions de rayonnement pendant le palpage aux rayons d'un objet 3 à partir de différents sens de projection,
    b) lecture des signaux électriques générés par l'ombre de rayonnement de l'objet (3) lors du palpage aux rayons et dérivés d'un récepteur de rayons (2) pendant la pause de rayonnement,
    c) acheminement de ces signaux à une unité de calcul et de commande (4) et
    d) calcul d'au moins un cliché de couche dans au moins une position de couche prédéfinissable en se basant sur ces signaux.

2. Procédé selon la revendication 1, dans lequel les impulsions de rayonnement sont générées par commande d'un générateur de rayons ou par commande d'un dispositif d'interruption des rayons.

3. Procédé selon la revendication 1 ou 2, dans lequel le palpage aux rayons a lieu avec une modification graduelle des sens de projection, dans lequel une impulsion de rayonnement est générée pour un premier sens de projection et

dans lequel, pendant le réglage dans un deuxième sens de projection, les signaux du récepteur de rayons (2) sont lus pendant la pause de rayonnement.

4. Procédé selon la revendication 3, dans lequel la vitesse du réglage d'une unité de prise de clichés composée de l'émetteur de rayons (1) et du récepteur de rayons (2) de l'appareil de diagnostic radiographique est plus faible pendant les impulsions de rayonnement que pendant la pause de rayonnement.

5. Procédé selon l'une quelconque des revendications 1 à 4, dans lequel le palpage aux rayons se fait par sections et dans lequel le calcul d'une image de couche pour une section est déjà réalisé pendant que le palpage aux rayons continue pour au moins une autre section.

6. Procédé selon l'une quelconque des revendications 1 à 5, dans lequel le récepteur de rayons (2) se présente comme un détecteur CCD, aSi, aSe ou CdTe.

7. Appareil de diagnostic radiographique pour la tomosynthèse numérique, comportant :

- un émetteur de rayons (1),
- un récepteur de rayons (2),
- un dispositif de réglage pour le réglage de l'émetteur de rayons (1) et du récepteur de rayons (2),
- ainsi qu'une unité de calcul et de commande (4) qui contrôle l'émetteur de rayons (1) et le dispositif de réglage, à laquelle sont communiqués les signaux électriques dérivés du récepteur de rayons (2) et qui est conçue pour calculer des clichés de couches,

**caractérisé en ce que** l'unité de calcul et de commande est conçue de manière à ce

- qu'elle contrôle l'émetteur de rayons de manière à ce que celui-ci génère des impulsions de rayonnement,
- qu'elle lise les signaux électriques dérivés du récepteur de rayons pendant la pause de rayonnement,
- et qu'elle calcule un cliché de couche dans au moins une position de couche prédéfinissable en se basant sur ces signaux.

FIG 1

FIG 2